(19) Europäisches Patentamt
European Patent Office
Office européen des brevets

(11) EP 2 440 551 B1

(12) **FASCICULE DE BREVET EUROPEEN**

(45) Date de publication et mention de la délivrance du brevet:
**06.08.2014 Bulletin 2014/32**

(21) Numéro de dépôt: **10724813.0**

(22) Date de dépôt: **08.06.2010**

(51) Int Cl.:
***C07D 407/04*** (2006.01) ***A61K 31/375*** (2006.01)
***A61P 29/00*** (2006.01)

(86) Numéro de dépôt international:
**PCT/EP2010/057979**

(87) Numéro de publication internationale:
**WO 2010/142663 (16.12.2010 Gazette 2010/50)**

(54) **MONO ESTERS ET BIS ESTERS D'ACIDE GRAS INSATURE SUR L'ACIDE ASCORBIQUE ET LEURS UTILISATIONS COSMETIQUES**

UNGESÄTTIGTE FETTSÄUREMONOESTER UND -DIESTER AUS ASCORBINSÄURE UND IHRE KOSMETISCHE VERWENDUNG

UNSATURATED FATTY ACID MONOESTERS AND DIESTERS ON ASCORBIC ACID AND COSMETIC USES THEREOF

(84) Etats contractants désignés:
**AL AT BE BG CH CY CZ DE DK EE ES FI FR GB GR HR HU IE IS IT LI LT LU LV MC MK MT NL NO PL PT RO SE SI SK SM TR**
Etats d'extension désignés:
**BA ME RS**

(30) Priorité: **08.06.2009 FR 0953785**

(43) Date de publication de la demande:
**18.04.2012 Bulletin 2012/16**

(73) Titulaire: **Pierre Fabre Dermo-Cosmétique**
**92100 Boulogne-Billancourt (FR)**

(72) Inventeurs:
- **POIGNY, Stéphane**
  **F-31600 Saubens (FR)**
- **DAUNES-MARION, Sylvie**
  **F-31000 Toulouse (FR)**
- **CASTEX RIZZI, Nathalie**
  **F-31770 Colomiers (FR)**
- **BOGDANOWICZ, Patrick**
  **F-31130 Balma (FR)**

(74) Mandataire: **Regimbeau**
**20, rue de Chazelles**
**75847 Paris Cedex 17 (FR)**

(56) Documents cités:
**EP-A1- 1 145 710** **FR-A- 2 580 644**
**US-A1- 2006 039 937** **US-A1- 2008 287 533**

Remarques:
Le dossier contient des informations techniques présentées postérieurement au dépôt de la demande et ne figurant pas dans le présent fascicule.

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 440 551 B1

## Description

La présente invention concerne des dérivés d'acide ascorbique ainsi que les compositions pharmaceutiques ou cosmétiques les contenant, leur procédé de préparation et des dérivés d'acide ascorbique pour leurs utilisations notamment en tant que médicament ou en tant que principe actif cosmétique.

L'acide ascorbique est un acide organique possédant des propriétés anti-oxydantes. Les sources naturelles de l'acide ascorbique sont les fruits et légumes frais et en particulier les agrumes.

Du fait de ses propriétés anti-oxydantes, l'acide ascorbique est souvent utilisé dans l'industrie agro-alimentaire comme conservateur sous le code E300 dans la liste des additifs alimentaires. L'acide ascorbique est également utilisé dans l'industrie cosmétique pour ses propriétés anti-radicalaires et kératolytiques reconnues.

L'acide ascorbique se présente sous forme d'une poudre blanche qui peut se colorer facilement à l'air ou en présence d'humidité.

Les acides gras insaturés correspondent à des acides gras présentant une ou plusieurs insaturations.

On parle d'acide gras mono-insaturé quand ils comprennent une seule insaturation. On parle d'acide gras polyinsaturé quand ils comprennent plusieurs insaturations.

Les acides gras insaturés peuvent en outre être d'origine végétale.

Les acides gras insaturés sont répartis en différentes classes. Ces classes sont définies par la position de la première insaturation en partant du côté opposé au groupe acide.

En particulier, on distingue trois classes principales d'acides gras insaturés : les omégas 3, les omégas 6 et les omégas 9. Les acides gras insaturés cis des séries omégas 3, omégas 6 et omégas 9 comprennent plusieurs acides gras dits essentiels. Ces acides gras sont dits essentiels car ils ne peuvent être apportés que par l'alimentation.

Les acides gras mono insaturés omégas 9 dont le constituant principal est l'acide oléique (C18 :1) sont connus pour avoir des effets bénéfiques concernant la prévention des maladies cardiovasculaires.

Les acides gras polyinsaturés (AGPI) appartenant à la classe des omégas 3 et des omégas 6 sont également reconnus pour avoir des effets préventifs concernant les maladies cardiovasculaires et le cancer. Il est notamment conseillé par l'AFSSAP de respecter un ratio entre les acides gras insaturés omégas 3 et omégas 6 dans l'alimentation, à savoir un ratio de un acide alpha linolénique pour 5 acides linoléiques.

Outre leurs effets métaboliques, ils sont capables de modifier l'expression de gènes codant pour des protéines intracellulaires. Ces effets géniques des AGPI s'effectueraient via des récepteurs nucléaires appelés PPARS (peroxysome proliferator activated receptor). Les PPARS appartiennent à la famille des récepteurs nucléaires hormonaux de type stéroïdien. Ils forment des hétérodimères avec les récepteurs RXR (Retinoic X Receptor) de l'acide rétinoïque et modulent l'expression de gènes. Ainsi, les AGPI $\omega 3$ seraient des régulateurs négatifs de la réponse inflammatoire, en inhibant la voie d'activation NF-$\kappa$B, par l'intermédiaire de l'induction de l'expression de I$\kappa$B$\alpha$ l'inhibiteur majeur de la voie NF-$\kappa$B (Ren J and Chung SH. J Agric Food Chem. 2007 55: 5073-80). De plus, les AGPI $\omega 3$ ont une action inhibitrice sur la synthèse de l'acide arachidonique au profit de la synthèse des acides docosahexanoïque et eicosapentanoïques (Calder PC. Lipids : 2001; 36, 1007-24).

Les acides gras insaturés en particulier les acides gras polyinsaturés sont connus pour leurs propriétés dermatologiques (Monpoint S, Guillot B, Truchetet F et al. Acides gras essentiels en dermatologie. Ann Dermatol Vénéréol, 1992, 119 : 233-239.). En particulier l'acide linoléique est un acide gras polyinsaturé qui intervient dans la fabrication de la membrane cellulaire. En effet, une carence en acide linoléique entraîne une sécheresse cutanée et la présence d'allergie.

Les composés selon l'invention sont des bis esters ou des mono-esters d'acide gras insaturé sur l'acide ascorbique dont les hydroxyles en position 5 et 6 sont protégés par un acétal cyclique et plus particulièrement un acétal cyclique de l'acétone (acétonide). Cette fonction est facilement hydrolysable en milieu légèrement acide, à savoir au niveau de la peau qui présente un pH acide entre 5,2 et 7. L'acétal cyclique va ainsi protéger l'acide ascorbique de l'oxydation de sa chaîne latérale. L'utilisation d'un acétal cyclique permet d'obtenir une structure d'une taille acceptable ayant une forte solubilité dans les phases grasses.

Les esters d'acides gras sont également facilement clivables par les estérases présentes dans la peau ce qui permet la libération des acides gras insaturés (Redoules, D., Tarroux, R., Assalit, M.F. and Perié, J.J. Caracterization and assay of five enzymatic activities in the stratum corneum using tape-stripping, Skin Pharmacol. Appl. Skin Physiol., 12, 182-192 (1999)). Le clivage des esters d'acide gras insaturé par les estérases présentes dans la peau permet alors une diffusion lente des principes actifs ce qui correspond au concept de drug delivery.

Dans le cas des composés de formule (I) selon la présente invention, l'action des estérases de la peau sur les liaisons esters conduit à la libération d'un acide gras insaturé et d'une molécule d'acide ascorbique dans le cas d'un mono-ester d'acide gras insaturé ; ou de deux acides gras insaturés et d'une molécule d'acide ascorbique via le mono ester en position 2 dans le cas d'un bis esters d'acide gras insaturé.

En particulier dans le cas des bis esters d'acide gras insaturé, l'action des estérases conduira d'abord à la formation d'un mono-ester d'acide gras insaturé et à la libération de l'acide ascorbique, puis à la libération de l'acide

ascorbique et d'un autre acide gras insaturé (schéma 1).

Schéma 1 : Exemple de réaction de clivage par les estérases de la peau.

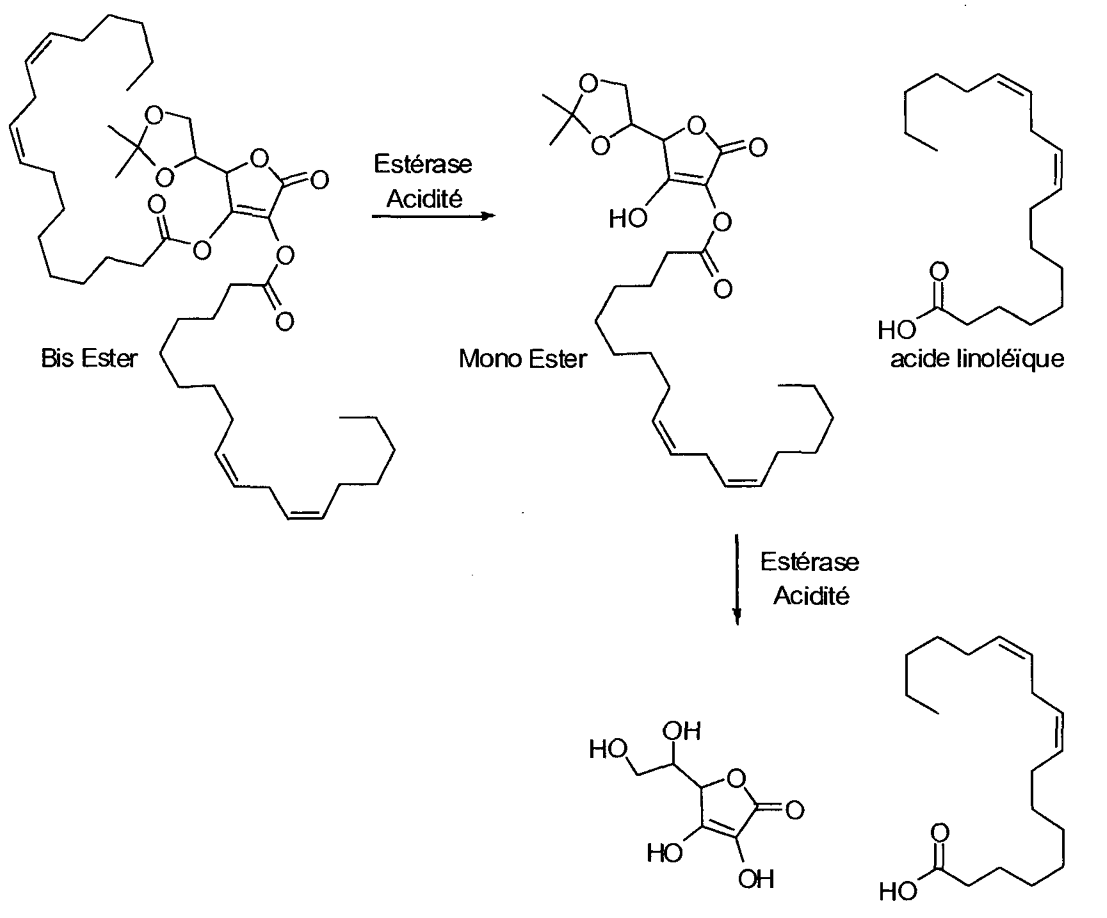


Bis Ester

Mono Ester

acide linoléïque

1 acide ascorbique

acide linoléïque

Ainsi la présente invention a pour objet un composé de la formule générale (I) suivante :

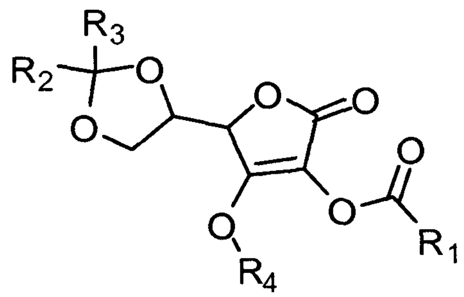

(I)

Pour laquelle :

- $R_1$ représente une chaîne hydrocarbonée provenant d'un acide gras insaturé de $C_{12}$ à $C_{24}$ comprenant au moins une insaturation, avantageusement 1 à 6 et de préférence 1 à 4 ; et
- $R_2$ et $R_3$ représentent indépendamment ou simultanément : un Hydrogène ou un $C_1$-$C_3$ alkyle ou un Phényle; et
- $R_4$ = un atome d'hydrogène ou $COR_1$', avec $R_1$' représente une chaîne hydrocarbonée provenant d'un acide gras insaturé de $C_{12}$ à $C_{24}$ comprenant au moins une insaturation, avantageusement 1 à 6 et de préférence 1 à 4.

Par « insaturation », on entend au sens de la présente invention une double liaison C=C.

Par « acide gras insaturé », on entend au sens de la présente invention un acide carboxylique ($R_1CO_2H$) ou ($R_1'CO_2H$) linéaire comprenant entre 12 et 24 atomes de carbone, de préférence de 14 à 18 atomes de carbone, et encore de préférence 18 atomes de carbone (incluant l'atome de carbone de la fonction acide carboxylique) et comprenant au moins une double liaison C=C, de préférence de 1 à 4 doubles liaisons C=C, ces doubles liaisons ayant de préférences une configuration cis.

Par « chaîne hydrocarbonée d'un acide gras insaturé », on entend, au sens de la présente invention, les chaînes hydrocarbonées ($R_1$) ou ($R_1'$) liées à la fonction acide de l'acide gras insaturé ($R_1CO_2H$) ou ($R_1'$ $CO_2H$). $R_1$ et $R_1'$ représentent donc une chaîne hydrocarbonée linéaire comprenant de 11 à 23, de préférence de 13 à 17, et encore de préférence 17, atomes de carbone et comprenant au moins une, de préférence 1 à 4, doubles liaisons C=C, ces doubles liaisons ayant de préférence une configuration *cis.* Selon l'invention, dans le cas où $R_4$ représente $COR_1'$, $R_1$ et $R_1'$ peuvent être identiques ou différents.

Les acides gras insaturés peuvent être l'acide lauroléïque ($C_{12:1}$), l'acide myristoléique ($C_{14:1}$), l'acide palmitoléïque ($C_{16:1}$), l'acide oléïque ($C_{18:1}$), l'acide ricinoléïque ($C_{18:1}$), l'acide gadoléïque ($C_{20:1}$) , l'acide érucique ($C_{22:1}$), l'acide $\alpha$-linolénique ($C_{18:3}$) , l'acide stéaridonique ($C_{18:4}$), l'acide eicosatriènoïque ($C_{20:3}$), l'acide eicosatetraènoïque ($C_{20:4}$). l'acide eicosapentaènoïque ($C_{20:5}$), l'acide docosapentaènoïque ($C_{22:5}$), l'acide docosahexaènoïque ($C_{22:6}$), l'acide tétracosapentaènoïque ($C_{24:5}$), l'acide tétracosahexaènoïque ($C_{24:6}$), l'acide linoléique ($C_{18:2}$), l'acide gamma-linolénique ($C_{18:3}$), l'acide eicosadiènoique ($C_{20:2}$), l'acide dihomo-gamma-linolénique ($C_{20:3}$), l'acide arachidonique ($C_{20:4}$), l'acide docosatétraènoïque ($C_{22:2}$), l'acide docosapentaènoïque ($C_{22:5}$), l'acide adrénique ($C_{22:4}$) et l'acide calendique ($C_{18:3}$).

Avantageusement, les composés selon l'invention sont ceux pour lesquels $R_1$ est un acide gras insaturé sélectionné parmi le groupe constitué par l'acide oléïque ($C_{18:1}$), l'acide linoléique ($C_{18:2}$), l'acide $\alpha$-linolénique ($C_{18:3}$) et l'acide $\gamma$-linolénique ($C_{18:3}$).

Avantageusement, les composés selon l'invention sont ceux dans lesquels lorsque $R_4$ représente $COR_1'$, $R_1'$ est un acide gras insaturé sélectionné parmi le groupe constitué par l'acide oléïque ($C_{18:1}$), l'acide linoléique ($C_{18:2}$), l'acide $\alpha$-linolénique ($C_{18:3}$) et l'acide $\gamma$-linolénique ($C_{18:3}$).

Par « alkyle », on entend au sens de la présente invention des chaînes hydrocarbonées aliphatiques linéaires ou ramifiées saturées et comprenant le nombre d'atomes de carbone spécifié. On peut par exemple citer, le méthyle, l'éthyle et le propyle.

Avantageusement, les composés selon l'invention sont ceux pour lesquels $R_2$ et $R_3$ représentent un $C_1$-$C_3$ alkyle.

Avantageusement, les composés selon l'invention sont ceux pour lesquels $R_2$ et $R_3$ représentent un méthyle.

Avantageusement, les composés selon l'invention sont ceux pour lesquels $R_1$ représente la chaîne hydrocarbonée d'un acide gras insaturé de $C_{14}$ à $C_{18}$ comprenant de 1 à 3 insaturation(s).

Avantageusement, les composés selon l'invention sont ceux pour lesquels, lorsque $R_4$ représente $COR_1'$, $R_1'$ représente la chaîne hydrocarbonée d'un acide gras insaturé de $C_{14}$ à $C_{18}$ comprenant de 1 à 3 insaturation(s).

Selon un mode de réalisation de l'invention, les composés de formule (I) sont ceux pour lesquels $R_4$ représente $COR_1'$.

Selon un autre mode de réalisation de l'invention, les composés de formule (I) sont ceux pour lesquels $R_4$ représente un atome d'hydrogène.

En particulier, les composés selon l'invention peuvent être choisis parmi les molécules suivantes :

1. <u>Molécule de formule générale (I) avec $R_4$ représente $COR_1'$</u>

- dioctadéca-9,12-diènoate de (9Z,9'Z,12Z,12'Z)-2-(2,2-diméthyl-1,3-dioxolan-4-yl)-5-oxo-2,5-dihydrofuran-3,4-diyle
- dioctadéca-9,12,15-triènoate de (9Z,9'Z,12Z,12'Z,15Z,15'Z)-2-(2,2-diméthyl-1,3-dioxolan-4-yl)-5-oxo-2,5-dihydrofuran-3,4-diyle
- dioctadéca-6,9,12-triènoate de (6Z,6'Z,9Z,9'Z,12Z,12'Z)-2-(2,2-diméthyl-1,3-dioxolan-4-yl)-5-oxo-2,5-dihydrofuran-3,4-diyle
- dioléate de (Z)-2-(2,2-diméthyl-1,3-dioxolan-4-yl)-5-oxo-2,5-dihydrofuran-3,4-diyle.

2. <u>Molécule de formule générale (I) avec $R_4$ représente un atome d'hydrogène</u>

- (9Z,12Z)-5-(2,2-dimethyl-1,3-dioxolan-4-yl)-4-hydroxy-2-oxo-2,5-dihydrofuran-3-yl octadeca-9,12-dienoate
- (9Z,12Z,15Z)-5-(2,2-dimethyl-1,3-dioxolan-4-yl)-4-hydroxy-2-oxo-2,5-dihydrofuran-3-yl octadeca-9,12,15-trienoate
- (6Z,9Z,12Z)-5-(2,2-dimethyl-1,3-dioxolan-4-yl)-4-hydroxy-2-oxo-2,5-dihydrofuran-3-yl octadeca-6,9,12-trienoate

- 5-(2,2-dimethyl-1,3-dioxolan-4-yl)-4-hydroxy-2-oxo-2,5-dihydrofuran-3-yl oleate.

La présente invention a également pour objet un composé de formule générale (I) pour son utilisation en tant que médicament.

La présente invention a également pour objet un composé de formule (I) générale pour son utilisation en tant que principe actif cosmétique.

En particulier, l'invention a également pour objet un composé de formule (I) générale pour son utilisation en tant que principe actif dépigmentant, principe actif anti-âge, principe actif anti-oxydant, principe actif hydratant, principe actif anti-inflammatoire, ou principe actif stimulant la repousse du poil et/ou du cheveu.

L'invention s'étend aussi à une composition pharmaceutique ou cosmétique caractérisée en ce qu'elle comprend au moins un composé de formule générale (I) en association avec un excipient pharmaceutiquement ou cosmétiquement acceptable adapté notamment à une administration percutanée.

Dans la présente invention, on entend désigner par « pharmaceutiquement ou cosmétiquement acceptable » ce qui est utile dans la préparation d'une composition pharmaceutique ou cosmétique, qui est généralement sûr, non toxique et ni biologiquement ni autrement non souhaitable et qui est acceptable pour une utilisation thérapeutique ou cosmétique, notamment par application topique.

Les compositions pharmaceutiques ou cosmétiques selon l'invention pourront se présenter sous les formes qui sont habituellement connues pour une administration topique sur la peau ou sur le cuir chevelu, c'est-à-dire notamment les lotions, les mousses, les gels, les dispersions, les émulsions, les shampooings, les sprays, les sérums, les masques, les laits corporels ou les crèmes, avec des excipients permettant notamment une pénétration cutanée afin d'améliorer les propriétés et l'accessibilité du principe actif. Les compositions pharmaceutiques ou cosmétiques selon l'invention pourront également se présenter sous la forme de gels injectables (en association par exemple avec de l'acide hyaluronique, du collagène, ou de l'alginate...) habituellement utilisés pour le comblement des rides.

Ces compositions contiennent généralement, outre le ou les composés selon la présente invention, un milieu physiologiquement acceptable, en général à base d'eau ou de solvant, par exemple des alcools, des éthers ou des glycols. Elles peuvent également contenir des agents tensioactifs, des agents complexant, des conservateurs, des agents stabilisants, des émulsifiants, des épaississants, des gélifiants, des humectants, des émollients, des oligo-éléments, des huiles essentielles, des parfums, des colorants, des agents matifiants, des filtres chimiques ou minéraux, des agents hydratants ou des eaux thermales, etc.

Ces compositions peuvent en outre contenir d'autres principes actifs conduisant à un effet complémentaire ou éventuellement synergique.

Avantageusement, les compositions selon la présente invention comprendront de 0,01% à 10% en poids, de préférence de 0,1% à 5% en poids du ou des composés de formule générale (I).

Ces compositions sont plus particulièrement destinées à la dépigmentation de la peau et/ou des cheveux et/ou des poils, au traitement et/ou à la prévention du vieillissement de la peau, à l'hydratation de l'épiderme, à la stimulation de la repousse du poil et/ou du cheveu, ou au traitement de l'inflammation de la peau.

La présente invention a également pour objet une méthode cosmétique de traitement et/ou de prévention du vieillissement de la peau.

L'objet de l'invention s'étend également à un procédé de blanchiment et d'éclaircissement de la peau humaine et/ ou des poils et/ou des cheveux par application d'une composition cosmétique contenant au moins un composé de formule générale (I).

La présente invention a également pour objet un procédé de préparation d'un composé de formule (I) par couplage d'un acide gras insaturé, dont la fonction carboxylique se trouve sous forme activée, et d'un dérivé d'acide ascorbique de formule (II).

Schéma 2 : Procédé de préparation d'un composé de formule (I)

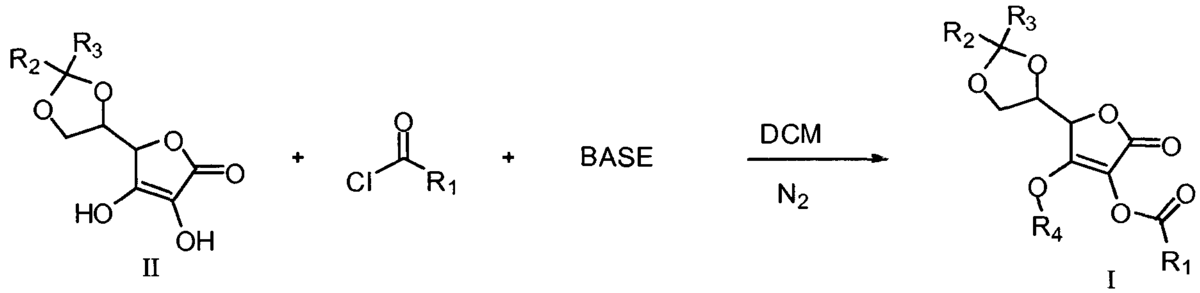


II

I

(DCM = dichlorométhane)

(Avec $R_4$ peut représenter $COR_1$' ou un atome d'hydrogène)

La réaction de couplage selon l'invention est réalisée en présence d'une base et éventuellement en présence d'un auxiliaire de couplage.

La base peut par exemple être la pyridine ou la triéthylamine.

L'auxiliaire de couplage peut par exemple être le 4-Diméthylaminopyridine.

Par « forme activée », on entend, au sens de la présente invention, une fonction acide carboxylique modifiée de manière à la rendre plus active vis-à-vis des nucléophiles. Ces formes activées sont bien connues de l'homme du métier et peuvent être en particulier un chlorure d'acide.

La présente invention sera mieux comprise à la lumière des exemples non limitatifs qui suivent.

EXEMPLE (1) : Synthèse des composés selon l'invention.

- Molécule de formule générale (I) avec $R_4$ représente $COR_1$' :

1.1 Procédé général A : à partir d'un chlorure d'acide gras insaturé et pyridine

1.1a Molécule 1 : dioctadéca-9,12-diènoate de (9Z,9'Z,12Z,12'Z)-2-(2,2-diméthyl-1,3-dioxolan-4-yl)-5-oxo-2,5-dihydrofuran-3,4-diyle-

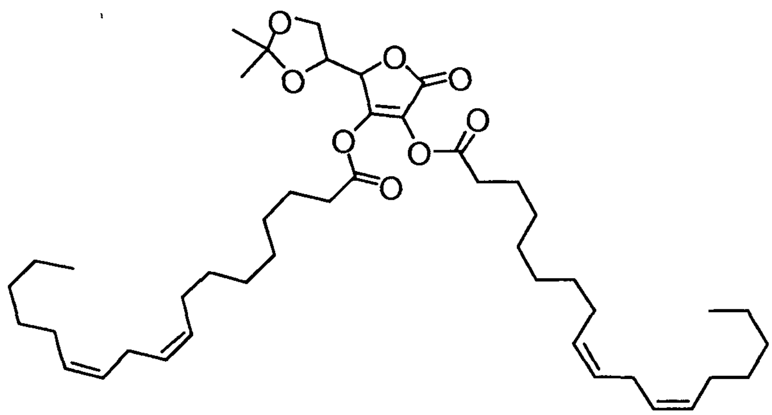

- Voie 1 : A une solution de l'acide 5,6-O-Isopropylidène-ascorbique (1,08g, 5 mmol, 1 éq.) et de 4-Diméthylaminopyridine (61 mg , 0,5 mmol, 0,1 éq.) dans 10 ml de pyridine anhydre et sous azote, sont ajoutés 2,9 g de chlorure de linoléoyle (10 mmol, 2 éq.) puis le mélange est agité à température ambiante pendant 16 heures. La réaction est suivie par CCM (chromatographie sur couche mince).

Après retour à température ambiante, le solvant est évaporé puis le résidu extrait avec un mélange éther/eau. La phase organique est lavée deux fois par une solution HCl 1N, puis par une solution saturée en NaCl. Après séchage sur sulfate de magnésium, une huile est obtenue après évaporation du solvant.

Le résidu est alors purifié sur silice par le mélange heptane/Acétate d'éthyle (100/0 à 70/30) ou par HPLC (chromatographie liquide haute performance) préparative. Le produit obtenu sous forme d'une huile incolore est séché sous vide pendant une nuit.

1,49 g sont alors obtenus avec un rendement de 40%.
RMN $^1$H (400 MHz, $CDCl_3$) : δ : 0,83 (2t, 6H) ; 1,27-1,39 (m, 34H) ; 1,68 (m, 4H) ; 2,06 (m, 8H) ; 2,53 (2t, 4H) ; 2,77 (2t, 4H); 4,01 (dd, 1H) ; 4,18 (dd, 1H) ; 4,35 (td, 1H) ; 5,14 (d, 1H) ; 5,35 (m, 8H).
RMN $^{13}$C (100 MHz, $CDCl_3$) : δ : 14,01; 22,6-33,6 (aliphatiques) ; 65,19; 72,9; 75,3; 76,7 ; 77; 110,8; 122,0; 127-130; 150,8 ; 167,7 ; 168, 169.

- Voie 2: l'acide 5,6-O-Isopropylidène-ascorbique (1g) est mis en suspension dans le dichlorométhane (19ml). La pyridine (0.79ml) est additionnée et le milieu réactionnel devient hétérogène blanc (T=15°C). Après 10 minutes sous agitation, le milieu réactionnel est refroidi à 0°C à l'aide d'un bain de glace. Une solution de chlorure de linoléoyle (2.98ml) dans le dichlorométhane (6ml) est alors additionnée sur une période de 5 minutes puis le bain de glace est retiré et le milieu réactionnel est laissé sous agitation 30 minutes.

Le milieu réactionnel est lavé par de l'eau (3 x 50ml), par une solution de sulfate de cuivre à 2% (p/v) (2 x 50ml) puis à nouveau par de l'eau (50ml). La phase organique est ensuite séchée sur sulfate de magnésium puis concentrée sous vide pour fournir une huile marron (3,3g ; Rendement 96%).
Cette huile est conservée sous atmosphère d'azote à -18°C.

1.2 Procédé général B : à partir d'un chlorure d'acide gras insaturé et triéthylamine

1.2.a Molécule 2 : dioctadéca-9,12,15-triènoate de (9Z, 9'Z, 12Z, 12'Z, 15Z, 15'Z)-2-(2,2-diméthyl-1,3-dioxolan-4-yl)-5-oxo-2,5-dihydrofuran-3,4-diyle -

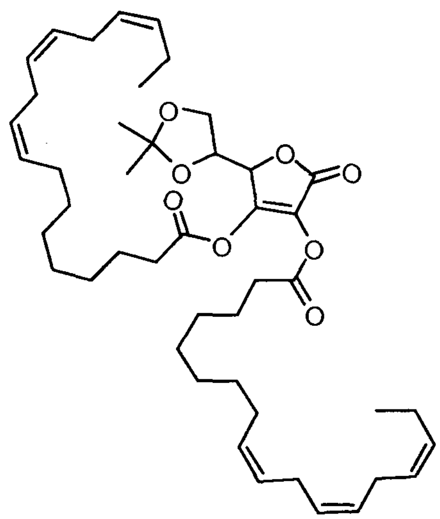

A une solution de l'acide 5,6-O-Isopropylidène-ascorbique (432 mg, 2 mmol, 1 éq.) et de triéthylamine (960 μl, 7 mmol, 3,5 éq.) dans 20 ml de dichlorométhane anhydre et sous azote, sont ajoutés 1,91 ml de chlorure de linolénoyle (6 mmol, 3 éq., 70% technique), puis le mélange est agité à température ambiante pendant 16 heures. La réaction est suivie par CCM.

Après retour à température ambiante, le solvant est évaporé puis le résidu extrait avec un mélange éther/eau. La phase organique est lavée deux fois par une solution HCl 1N puis par une solution saturée en NaCl. Après séchage sur sulfate de magnésium, une huile est obtenue après évaporation du solvant. Le résidu est alors purifié sur silice par le mélange heptane/Acétate d'éthyle (100/0 à 70/30) ou par HPLC préparative. Le produit obtenu sous forme d'une huile incolore est séché sous vide pendant une nuit.
604 mg sont alors obtenus avec un rendement de 41%.
RMN $^1$H (400 MHz, $CDCl_3$): δ : 0, 91 (2t, 6H) ; 1,27-1,39 (m, 30H) ; 1,67 (m, 4H) ; 2,06 (m, 4H) ; 2,53 (2t, 4H) ; 2,79 (2t, 4H) ; 4,09 (dd, 1H) ; 4,18 (dd, 1H) ; 4,36 (td, 1H) ; 5,14 (d, 1H) ; 5,38 (m, 12H).

1.2.b Molécule 3 : dioctadéca-6,9,12-triènoate de (6Z, 6'Z, 9Z, 9'Z, 12Z, 12'Z)-2-(2,2-diméthyl-1,3-dioxolan-4-yl)-5-oxo-2,5-dihydrofuran-3,4-diyle-

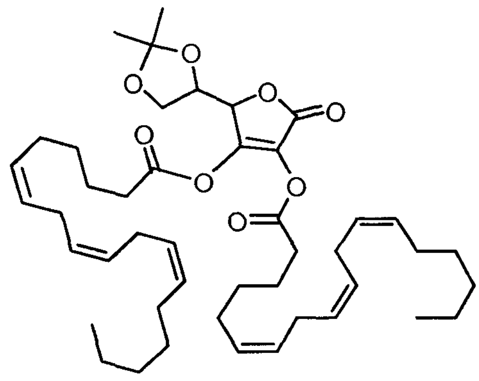

A partir de 2,5 équivalents de chlorure de gamma linolénoyle (98%).
Huile incolore avec un rendement de 68%.
RMN $^1H$ (400 MHz, $CDCl_3$) : $\delta$: 0,89 (2t, 6H) ; 1, 27-1, 44 (m, 20H) ; 1,47 (m, 4H) ; 1,68 (m, 4H) ; 2,06 (m, 8H) ; 2,55 (m, 4H) ; 2,80 (m, 8H); 4,01 (dd, 1H) ; 4,18 (dd, 1H) ; 4,36 (td, 1H) ; 5,14 (d, 1H) ; 5,37 (m, 12H).

1.2.c Molécule 4 dioléate de (Z)-2-(2,2-diméthyl-1,3-dioxolan-4-yl)-5-oxo-2,5-dihydrofuran-3,4-diyle -

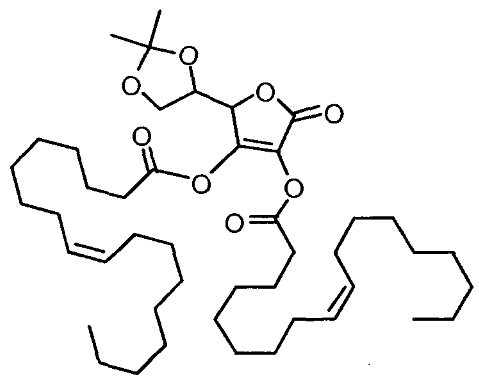

A partir de 2,5 équivalents de chlorure d'oléoyle (98%) Huile incolore avec un rendement de 53% après purification.
RMN $^1H$ (400 MHz, $CDCl_3$) : $\delta$ : 0,88 (2t, 6H) ; 1,26-1,55 (m, 46H) ; 1,67 (m, 4H) ; 2,00 (m, 8H) ; 2,53 (m, 4H) ; 2,77 (2t, 4H); 4,09 (dd, 1H) ; 4,18 (dd, 1H) ; 4,36 (td, 1H) ; 5,14 (d, 1H) ; 5,34 (m, 4H).

EXEMPLE (2) : Synthèse des composés selon l'invention.

- Molécule de formule générale (I) avec $R_4$ représente un atome d'hydrogène

2.1 Molécule 5 (9Z,12Z)-5-(2,2-dimethyl-1,3-dioxolan-4-yl)-4-hydroxy-2-oxo-2,5-dihydrofuran-3-yl octadeca-9,12-dienoate -

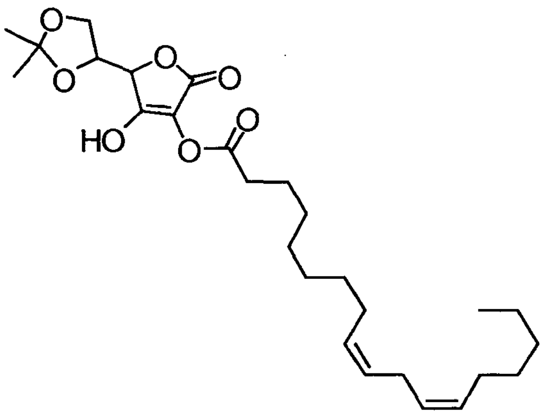

Dans un tricol sous azote est préparée une suspension de l'acide 5, 6-O-Isopropylidène-ascorbique (5,5g ; 25,44 mmol) dans 110 mL d'acétone puis 3,53 mL de triethylamine sont ajoutés. Formation d'un précipité blanc abondant. Le chlorure de linoléoyle (3,55 mL ; 11,06 mmol) est alors ajouté goutte à goutte sur 3 minutes puis le mélange est agité 10 minutes à température ambiante. Le mélange redevient limpide puis un petit précipité de chlorure de triéthylamonium se forme. Le solide est filtré sur fritté puis le filtrat tiré à sec pour obtenir une huile. Cette huile est reprise dans 100 mL d'acétate d'éthyle et lavée 3 fois avec une solution saturée de NaCl. La phase organique est séchée sur sulfate de magnésium, filtrée et évaporée pour conduire à 4,76 g d'un solide brun avec un rendement de 90%.
RMN $^1H$ (300 MHz, $CDCl_3$) : $\delta$ : 0,90 (t, 3H) ; 1,27-1,39 (m, 20H) ; 1,70 (m, 2H) ; 2,06 (m, 4H) ; 2,58 (t, 2H) ; 2,78 (t, 2H); 4,13 (dd, 1H) ; 4,20 (dd, 1H) ; 4,43 (m, 1H) ; 4,65 (d, 1H) ; 5,37 (m, 4H).
$[M+Na]^+$= 501,2 ; $[2M+Na]^+$= 979,7
$[M-H]^-$= 477,3 ; $[2M-H]^-$= 955,7

2.2 Molécule 6 5-(2,2-dimethyl-1,3-dioxolan-4-yl)-4-hydroxy-2-oxo-2,5-dihydrofuran-3-yl oleate -

Même mode opératoire que molécule 5 à partir de chlorure d'oléoyle.

Solide blanc, Rendement : 96%

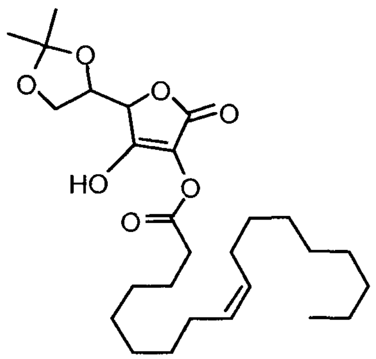

RMN $^1H$ (300 MHz, $CDCl_3$) : $\delta$ : 0,89 (t, 3H) ; 1, 28-1, 42 (m, 26H) ; 1, 69 (m, 2H) ; 2, 02 (m, 4H) ; 2, 62 (m, 2H) ; 4, 15 (dd, 1H) ; 4, 22 (dd, 1H) ; 4,45 (td, 1H) ; 4,70 (d, 1H) ; 5,36 (m, 2H).
$[M+Na]^+$= 503,2
$[M-H]^-$= 479,3

2.3 Molécule 7

(9Z, 12Z, 15Z)-5-(2,2-dimethyl-1,3-dioxolan-4-yl)-4-hydroxy-2-oxo-2,5-dihydrofuran-3-yl octadeca-9,12,15-trienoate -

Même mode opératoire que molécule 5 à partir de chlorure de linolénoyle.

Solide brun, Rendement : 91%

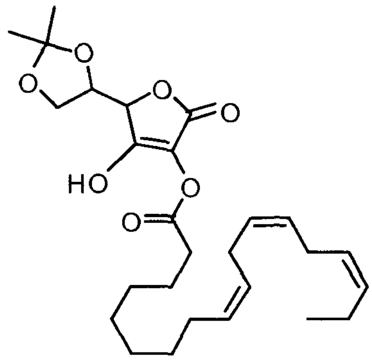

RMN $^{1}H$ (300 MHz, $CDCl_3$) : $\delta$ : 0,99 (t, 3H) ; 1, 30-1, 42 (m, 14H) ; 1,72 (m, 2H) ; 2,08 (m, 4H) ; 2,62 (m, 2H) ; 2,82 (m, 4H) ; 4,15 (dd, 1H) ; 4,22 (dd, 1H) ; 4,45 (td, 1H) ; 4,70 (d, 1H) ; 5,38 (m, 6H).
$[M+Na]^+$= 499,1
$[M+H]^+$= 477,2

EXEMPLE (3) : Composition selon l'invention

1. Une crème

| Ingrédients (Noms commerciaux) | Désignation INCI | Pourcentage | Fonction |
|---|---|---|---|
| Eau purifiée | Eau | QSP* 100% | |
| Glycérine | Glycérine | 3 | Humectant |
| EDTA**, 2Na | EDTA disodique | 0,1 | Agent complexant |
| Phénoxyéthanol | Phénoxyéthanol | 0,35 | Conservateur |
| Sepiplus™ 400 | Polyacrylate-13 et Polyisobutène et Polysorbate 20 et eau | 1 | Gélifiant, agent stabilisant |
| Simulsol™ 165 | Stéarate de glycéryl et stéarate de PEG*** 100 | 4 | Emulsionnant |
| Lanette ® 16 | Alcool cétylique | 1 | Facteur de consistance |
| Myritol ® 318 | Triglycérides caprique/caprilique | 6 | Emollient |
| Primol ® 352 | Paraffine liquide | 4 | Emollient |
| Cetiol ® CC | Carbonate de dicaprylyle | 4 | Emollient |

(suite)

| Ingrédients (Noms commerciaux) | Désignation INCI | Pourcentage | Fonction |
|---|---|---|---|
| molécule 1 | 9Z,9'Z,12Z,12'Z)-2-(2,2-diméthyl-1,3-dioxolan-4-yl)-5-oxo-2,5-dihydrofuran-3,4-diyl dioctadéca-9,12-diènoate | 0,5 | Actif |
| Chlorphénésine | Chlorphénésine | 0,27 | Conservateur |
| Micropearl M100 | Polyméthacrylate de méthyle | 1 | Poudre |
| Parfum | Fragrance | 0,1 | Parfum |
| *QSP : Quantité suffisante pour<br>*EDTA : acide éthylène diamine tétraacétique<br>***PEG : polyéthylène glycol | | | |

2. Un lait

| Ingrédients (Noms commerciaux) | Désignation INCI | Pourcentage | Fonction |
|---|---|---|---|
| Eau purifiée | Eau | QSP* 100% | |
| Glycérine | Glycérine | 3 | Humectant |
| EDTA**, 2 Na | EDTA disodique | 0,1 | Agent complexant |
| Phénoxyéthanol | Phénoxyéthanol | 0,35 | Conservateur |
| Synthalen ® K | Carbomère | 0,2 | Gélifiant, agent stabilisant |
| Pemulen ® TR1 | un copolymère de l'acide acrylique et du méthacrylate alkylique | 0,3 | Gélifiant, agent stabilisant |
| Myritol ® 318 | Triglycéride caprique/caprilique | 4 | Emollient |
| Primol ® 352 | Paraffine liquide | 4 | Emollient |
| molécule 1 | 9Z,9'Z,12Z,12'Z)-2-(2,2-diméthyl-1,3-dioxolan-4-yl)-5-oxo-2,5-dihydrofuran-3,4-diyl dioctadéca-9,12-diènoate | 0,5 | Actif |
| Chlorphénésine | Chlorphénésine | 0,27 | Conservateur |
| Parfum | Fragance | 0,1 | Parfum |
| *QSP : Quantité suffisante pour<br>**EDTA : acide éthylène diamine tétraacétique | | | |

EXEMPLE (4) : Résultats des tests biologiques

4.1 Cibles des tests biologiques

La matrice extracellulaire (MEC) est une structure dynamique ayant un rôle structurel et régulateur pour les tissus. La MEC du derme est constituée de fibres (collagènes et élastine) et de substance fondamentale (eau, sels minéraux, glycoprotéines, acide hyaluronique et protéoglycanes). Elle confère à la peau sa turgescence et ses propriétés mécaniques: fermeté, élasticité et tonicité. La MEC subit en permanence un remaniement lié à un équilibre entre la

synthèse et la dégradation des macromolécules qui la constituent. La MEC est constituée de quatre types de macromolécules: les collagènes, l'élastine, les glycoprotéines de structure et les glycosaminoglycanes (tel que l'acide hyaluronique).

Les collagènes sont les protéines fibreuses très abondantes dans le derme. On y retrouve en majorité les collagènes fibrillaires, de type I, III, et V. Les collagènes constituent le composant essentiel du réseau fibreux et jouent un rôle mécanique, conférant résistance et élasticité à la peau.

La dégradation de la MEC est mise en place au cours de certains processus physiologiques (cicatrisation, développement embryonnaire, angiogénèse...) mais aussi au cours de processus pathologiques (arthrite, arthrose, athérosclérose, progression tumorale et formation de métastases...) (Fisher et al, N. Engl. J. Med. 333: 1419, 1997; Shapiro SD Current Opinion in Cell Biology 10 : 602, 1998). Les composants de la MEC sont majoritairement dégradés par des enzymes du type endopeptidases appelés métalloprotéinases matricielles ou MMPs (Nagase and Woessner, J. Biol. Chem. 274 : 21491, 1999). Les MMPs participent activement au processus de cicatrisation mais elles contribuent aussi au relâchement cutané et à l'apparition des rides. Les MMPs sont des enzymes du type endopeptidases à zinc.

La MMP-1, ou collagénase interstitielle, dégrade la triple hélice des collagènes fibrillaires de type I majoritairement, et dégrade également les collagènes II, VII, VIII et X (Kähari VM and Saarialho-Kere U, Exp. Dermatol. 6: 199,1997). La MMP-1 a donc un rôle crucial dans l'initiation de la dégradation des collagènes. Cette activité collagénolytique est aussi associée à la cicatrisation. La stromélysine de type 1 (MMP-3) dégrade les glycoprotéines telles que la fibronectine et la laminine, certains protéoglycanes, l'élastine, la gélatine et les collagènes IV et V. Au niveau cutané, les MMP-1 et MMP-3 sont exprimées à la fois par les kératinocytes et les fibroblastes. Au cours du vieillissement cutané, une diminution de la quantité d'acide hyaluronique est également mesurée, aussi bien au niveau épidermique que dermique. Cette diminution est la résultante d'une diminution de la synthèse d'acide hyaluronique et d'une augmentation de l'activité hyaluronidase (Stern R, Maibach HI (2008)Hyaluronan in skin: aspects of aging and its pharmacologic modulation. Clin Dermatol 26:106-22).

Par ailleurs, de nombreux travaux mettent en évidence une augmentation de cytokines pro-inflammatoires (IL-6, IL-8...) au cours du vieillissement (Franceschi et al, Mechanisms of ageing and development, 92, 2006) Ce contexte pro-inflammatoire ou "inflammaging" serait directement impliqué dans l'apparition de certains signes du vieillissement, notamment au niveau cutanée (Mocchegiani et al., Biomed Central, 1 : 5, 2004; Licastro et al., Neurobiol. Aging, 2006). Les interleukines, cytokines produites par les lymphocytes T, sont alors exprimées (IL-6 ou IL-8).

Pour mettre en évidence l'activité des composés de formule générale (I) contre le vieillissement cutané et contre l'inflammation, l'effet de ces composés a été mesuré sur l'expression génique de la MMP-1, de la MMP-3 de l'IL8 dans des fibroblastes dermiques humains traités à l'$H_2O_2$, mimant ainsi le processus de sénescence cellulaire. L'effet de ces composés de formule générale (I) a également été mesuré sur la synthèse d'acide hyaluronique et sur l'activité hyaluronidase. La stimulation de la synthèse de l'acide hyaluronique permet également d'améliorer et/ou de restaurer l'hydratation cutanée (Bissett DL. J Cosmet Dermatol. 2006 Dec;5(4):309-15. Glucosamine: an ingredient with skin and other benefits).

De la même façon, pour mettre en évidence l'activité dépigmentante des composés de formule générale (I) selon l'invention, l'effet de ces composés a été mesuré sur la synthèse de mélanine par dosage colorimétrique sur une lignée cellulaire de mélanomes murins: la lignée B16-F10.

De plus, l'effet des composés de formule générale (I) sur la repousse des cheveux et/ou des poils a également été évalué en mesurant leur activité sur la stimulation de la prolifération des cellules de la papille dermique et la stimulation de la croissance du follicule pileux humain.

4.2.1 Protocole du test concernant l'expression génique de la MMP-1, de la MMP-3 et de l'IL8 dans les fibroblastes humains

- Traitement des cellules

Les fibroblastes dermiques humains (isolés de déchets opératoires cutanés) sont cultivés dans du milieu de culture DMEM + 10% SVF. Les cellules sont pré-traitées avec la vitamine C et le composé à tester pendant 16h à 37°C, puis stimulées avec $H_2O_2$. Les cellules sont ensuite replacées dans du DMEM avec la vitamine C et le dioctadéca-9,12-diènoate de (9Z,9'Z,12Z,12'Z)-2-(2,2-diméthyl-1,3-dioxolan-4-yl)-5-oxo-2,5-dihydrofuran-3,4-diyle. 72 heures après la fin du stress, les fibroblastes sont rentrés en sénescence: les fibroblastes sénescents ne prolifèrent plus, mais restent métaboliquement actifs (Hayflick, J Invest Dermatol., 73: 8-14, 1979). Les cellules sont alors lysées avec le tampon de lyse du kit RNeasy® (QIAGEN).

- Transcription inverse et PCR en temps réel

Les ARNs extraits sont transformés en ADN complémentaires en suivant les indications du kit Quantitect

Reverse Transcription de Qiagen. Les analyses de PCR en temps réel sont effectuées à l'aide du thermocycleur à fluorescence Icycler iQ (BIO-RAD)

Le niveau d'expression des ARNm codant pour MMP-1, MMP-3, IL8, a été analysé par la technique de PCR en temps réel. Pour normaliser le niveau d'expression du gène d'intérêt, la stratégie d'analyse développée par Vandesompele et al, Genome Biol. 3: RESEARCH0034 (2002) a été choisie. A partir des valeurs $\Delta C_T$, obtenues lors d'une première PCR, l'algorithme du logiciel Genorm version 3.4 (Vandesompele J et al, Genome Biol. 3: RESEARCH0034 2002) compare le niveau de stabilité de trois gènes de référence dans les conditions expérimentales précises du test cellulaire et permet de déterminer le gène de référence le plus stable. Dans notre modèle les trois gènes de référence testés sont les suivants : β-actine (Human β-actine), GAPDH (Human glyceraldehyde-3-phosphate deshydrogenase), et YWHAZ (Tyrosine-3-monooxygenase, tryptophane-5-monooxygenase activation protein zeta plolypeptide).

La première étape consiste à normaliser les valeurs des Ct obtenues pour le gène d'intérêt par rapport aux valeurs des Ct obtenues pour le gène de référence, et ce pour chaque condition expérimentale.

Ainsi est calculé:

$$\Delta Ct = Ct_{\text{gène intérêt}} - Ct_{\text{gène référence}}$$

Ces ΔCt représentent les valeurs brutes non transformées utilisées pour l'analyse statistique.

La deuxième étape du calcul consiste à déterminer la variation du nombre de copies du gène d'intérêt lors du traitement. Pour cela, le ΔΔCt est calculé :

$$\Delta\Delta Ct = \Delta Ct_{\text{FNH non traité}} - \Delta Ct_{\text{FNH traité}}$$

Pour le témoin non traité, cette QR est donc égale à 1. Il est ensuite possible de calculer un facteur d'induction ou d'inhibition du gène d'intérêt par rapport à ce témoin.

4.2.2 Résultats du test concernant l'expression de la MMP-1, de la MMP-3 et de l'IL8 dans les fibroblastes humains

- Analyse statistique

L'analyse statistique est réalisée selon un test appelé « one-way ANOVA ». L'analyse de variance par le test de Dunnett permet ensuite de comparer, pour chacun des gènes analysés, les ΔCt des Fibroblastes normaux adultes par les composés en présence de $H_2O_2$ dans les deux cas. Ce test donne alors les valeurs des « p value » caractérisant la significativité des résultats obtenus pour les deux conditions. Le degré de significativité est établi comme suit :

- significatif pour p < 0,05 (*)
- très significatif pour p < 0,005 (**)
- hautement significatif pour p < 0,001 (***)
- non significatif pour p > 0,05

- Analyse de l'expression des ARNm de MMP-1, MMP-3 et IL-8 dans les fibroblastes dermiques humains sénescents traités ou non avec les composés de l'invention

→ Analyse de la quantité relative des ARNm

Les fibroblastes humains dermiques sont incubés en présence d'acide ascorbique ou de dioctadéca-9,12-diènoate de (9Z,9'Z,12Z,12'Z)-2-(2,2-diméthyl-1,3-dioxolan-4-yl)-5-oxo-2,5-dihydrofuran-3,4-diyle (= actif 1 dans le tableau 2) avant et après avoir été stimulés par l'$H_2O_2$. Afin d'analyser l'effet de ces extraits sur l'expression des ARNm modulés par l'$H_2O_2$, les échantillons traités avec les actifs et stimulés à l'$H_2O_2$ sont comparés avec l'échantillon stimulé uniquement à l'$H_2O_2$. Pour chaque échantillon, le niveau d'expression de l'ARNm d'intérêt doit être normalisé par le niveau d'expression de l'ARNm du gène de référence le plus stable.

→ Analyse du pourcentage d'activité

Il est également possible de calculer des pourcentages d'inhibition du niveau d'expression des différents ARNm par les actifs à évaluer, selon la formule :

$$\% \text{ d'inhibition} = \frac{(QR^{H2O2} - QR^{non\ traité}) - (QR^{H2O2+actif} - QR^{non\ traité})\ x\ 100}{QR^{H2O2} - QR^{non\ traité}}$$

Tableau 1 : Quantité relative des ARNm de MMP-1, MMP-3 et IL-8 des fibroblastes jeunes ou sénescents et pourcentage d'inhibition du niveau d'expression des ARNm par les actifs testés.

| MMP1 | | |
|---|---|---|
| | QR | % Inhibition |
| $H_2O_2$ | 3.6 | |
| Acide ascorbique (3μM) | 3.2 | 18 |
| molécule 1 (3μM) | 0.9 | 105*** |
| | | |
| MMP3 | | |
| | QR | % Inhibition |
| $H_2O_2$ | 5.9 | |
| Acide ascorbique (3μM) | 3.5 | 50* |
| molécule 1 (3μM) | 1.9 | 81*** |
| | | |
| IL8 | | |
| | QR | % Inhibition |
| $H_2O_2$ | 3.1 | |
| Acide ascorbique (3μM) | 1.2 | 92*** |
| molécule 1 (3μM) | 1.0 | 102*** |

La molécule 1 (dioctadéca-9,12-diènoate de (9Z,9'Z,12Z,12'Z)-2-(2,2-diméthyl-1,3-dioxolan-4-yl)-5-oxo-2,5-dihydrofuran-3,4-diyle) inhibe de façon plus importante l'expression de MMP1, de MMP3 et de l'IL-8 que l'acide ascorbique.

La présente invention concerne des composés de formule générale (I) pour prévenir et/ou traiter le vieillissement cutané.

La présente invention s'étend également à des composés de formule (I) pour traiter les réactions inflammmatoires cutanées.

4.3.1 Protocole du test de dosage de la mélanine dans les molécules B16-F10

- Principe :

Il s'agit d'un test de mesure de la synthèse de mélanine par dosage colorimétrique sur une lignée cellulaire de mélanomes murins: la lignée B16-F10. Ce test permet d'évaluer le pouvoir dépigmentant de principes actifs.

Les cellules B16-F10 sont ensemencées dans des plaques 96 puits dans du milieu DMEM supplémenté de SVF (sérum de veau foetal), et incubées 24 heures à 37°C, 5%$CO_2$. Les cellules sont ensuite stimulées à l'$\alpha$-MSH (pour stimuler la synthèse de mélanine, la stimulation observée est d'environ 150%) et traitées 72 heures avec les actifs à tester. Chaque concentration d'actif est testée au moins en triplicat. La mélanine totale suivie de la mélanine intracellulaire dissoute dans du tampon de lyse sont alors dosées par lecture d'absorbance à 405 nm. Les protéines totales sont dosées dans le lysat et les résultats sont exprimés en mg mélanine / mg protéines. Le pourcentage d'activité est calculé comme suit :

$$-\%\ d'activit\acute{e} = \frac{\text{Moyenne normalisée du témoin} - \text{Moyenne normalisée du traité}}{\text{Moyenne normalisée du témoin}} \times 100$$

Une valeur négative indique une inhibition, alors qu'une valeur positive indique une induction de la synthèse de mélanine.

4.3.2 Résultats du test de dosage de la mélanine dans les cellules B16-F10

Tableau 2 : Pourcentage d'inhibition de la Mélanine intracellulaire et valeurs $IC_{50}$.

| Gamme | 100μM | 50μM | 20μM | 10μM | $IC_{50}$μM |
|---|---|---|---|---|---|
| Vitamine C | -22 | -5 | -7 | 5 | nd* |
| molécule 1 | -71 | -41 | -21 | -9 | 67 |
| molécule 2 | nd | nd | -53 | -11 | 20 |
| molécule 3 | nd | nd | -50 | -25 | 20 |
| molécule 4 | nd | -44 | -6 | 16 | 53 |
| molécule 5 | -78 | -35 | -13 | -4 | 66 |
| molécule 6 | -49 | -7 | nd | nd | 105 |
| *nd = non déterminé | | | | | |

Les résultats de ces tests (Tableau 2) démontrent que l'inhibition de la synthèse de mélanine intracellulaire par les molécules 1, 2, 3, 4, 5, 6 est supérieure à celle de la vitamine C.

La présente invention concerne également des composés de formule générale (I) pour dépigmenter la peau et/ou les poils et/ou les cheveux.

4.4.1 Protocole de dosage de la synthèse d'acide hyaluronique

Les kératinocytes HaCaT sont cultivés dans du milieu de culture DMEM + 10% SVF. Les cellules sont traitées avec les actifs à tester (Tableau I) pendant 48h à 37°C. L'acide hyaluronique (AH) synthétisé est dosé dans le milieu de culture à l'aide d'un kit de type ELISA (Echelon®).

Le pourcentage d'activité est déterminé comme suit:

- pourcentage de variation par rapport au contrôle:

$$\%p/C = ([AH]_{actif} / [AH]_{contrôle} \times 100) - 100$$

4.4.2 Résultat du test de dosage de l'acide hyaluronique sur la lignée de kératinocytes humains HaCaT

Tableau 3 : Pourcentage de stimulation de la synthèse d'acide hyaluronique.

| Concentration | 2μM | 20μM | 30μM | 300μM |
|---|---|---|---|---|
| Vitamine C | * | * | 9 | 19 |

(suite)

| Concentration | 2$\mu$M | 20$\mu$M | 30$\mu$M | 300$\mu$M |
|---|---|---|---|---|
| molécule 1 | 132 | 70 | * | * |
| *nd = non déterminé | | | | |

Les résultats de ces tests (Tableau 3) démontrent que la vitamine C, utilisée à des concentrations biologiquement actives (30 et 300$\mu$M), n'a pas d'effet sur la synthèse d'acide hyaluronique chez les kératinocytes HaCaT.
La molécule 1, à 2 et 20 $\mu$M, stimule fortement la synthèse d'acide hyaluronique: +132% + 70%.
La présente invention concerne donc des composés de formule générale I pour la prévention et/ou le traitement du vieillissement cutané. L'invention s'étend également à des composés de formule générale I pour améliorer et /ou restaurer l'hydratation cutanée (Bissett DL. J Cosmet Dermatol. 2006 Dec; 5(4):309-15. Glucosamine: an ingredient with skin and other benefits).

4.5.1 Protocole de dosage de l'activité hyaluronidase :

L'activité hyaluronidase est mesurée par dosage d'acide hyaluronique (HA) résiduel. Le substrat est fixé à un support puis mis en présence d'enzyme ainsi qu'une quantité de composé à tester. L'HA résiduel c'est-à-dire l'HA non hydrolysé est dosé grâce à un système de détection HA-Elisa : Hyaluronan enzyme-linked immunosorbent assay (Echelon Biosciences ®).
0.5 Unités de Hyaluronidase d'origine bovine (BTH, bovin testis hyaluronidase) sont préincubées à 37°c avec le composé à tester. Ce mélange est déposé sur l'HA fixé à la surface d'un puit d'une microplaque. La réaction enzymatique se fait à pH 7,2 à 37°c. Après lavage, il y a une étape de reconnaissance de l'HA résiduel par la hyaluronectine (NH). La quantité de NH liée est mesurée par ELISA avec un anticorps anti-NH conjugué à l'alcaline phosphatase. Le susbtrat p-nitrophenyl phosphate disodium salt (pnNpp) de la phosphatase permet une détection spectrophotométrique à 405nm. (Delpech B and coll, Analytical Biochemistry 149, 555-565 (1985) ; Robert Stern and coll. Analytical Biochemistry 251, 263-269 (1997)). L'intensité de DO est corrélée à la quantité d'HA résiduel. La quantité d'HA résiduel est directement liée à l'activité enzymatique. En présence d'inhibiteur, plus la quantité d'HA résiduelle est grande, plus l'échantillon est inhibiteur. Un pourcentage d'inhibition de la hyaluronidase sera exprimé.
Ainsi, sont calculées des activités nettes enzymatiques qui représentent la différence de l'activité brute moyenne sans enzyme et de l'activité brute de l'enzyme plus ou moins le composé à tester.
Le pourcentage d'inhibition enzymatique liée au composé à tester est calculé selon :

% inhibition = (Activité nette enzymatique maximale - Activité nette enzymatique en présence du composé à tester) / Activité nette enzymatique maximale

Où l'activité nette enzymatique maximale représente l'activité nette moyenne enzymatique de 0.5U.

4.5.2 Résultat du test de dosage de l'activité hyaluronidase

Tableau 4 : Mesure de l'inhibition enzymatique à différentes doses (moyennes en pourcentage)

| Concentration | molécule 1 | molécule 4 | molécule 5 | molécule 6 | 6-Ascorbyl palmitate | Acide Ascorbique |
|---|---|---|---|---|---|---|
| 500$\mu$M | 122 | * | * | * | * | 4 |
| 250$\mu$M | 119 | * | * | * | 112 | * |
| 200$\mu$M | * | * | * | * | 79 | * |
| 150$\mu$M | * | * | * | * | * | * |
| 100$\mu$M | 113 | * | * | * | 73 | 1 |
| 50$\mu$M | 76 | 111 | 76 | 106 | 71 | * |

(suite)

| Concentration | molécule 1 | molécule 4 | molécule 5 | molécule 6 | 6-Ascorbyl palmitate | Acide Ascorbique |
|---|---|---|---|---|---|---|
| 40µM | 86 | * | * | * | * | * |
| 30µM | 66 | * | * | * | * | * |
| 25µM | 46 | * | * | * | 57 | * |
| 20µM | 44 | * | * | * | * | * |
| 10µM | 21 | 72 | 31 | 34 | 31 | * |
| 5µM | 14 | * | * | * | * | * |
| * Non déterminé | | | | | | |

Les résultats de ces tests (Tableau 4) démontrent que la vitamine C, utilisée à ces concentrations (100 et /500uM), n'a pas d'effet sur la hyaluronidase (BTH).

Les résultats de ces tests démontrent que le 6-ascorbyl palmitate inhibe de façon intéressante la hyaluronidase de 10 à 250 µM avec effet dose.

Les molécules 1,4,5 et 6 de 5µM à 500 µM, inhibent fortement l'activité hyaluronidase : la molécule 1 présente un effet dose et son IC 50 se situe aux <u>alentours de 25 à 30 µM.</u>

Les molécules 1,4,5 et 6 sont équivalentes ou plus actives que l'ascorbyl palmitate qui est considérée comme la molécule de référence (A. Botzki and coll. JBC vol 279 N°44, pp45990-450007)

Les résultats de ces tests démontrent que l'inhibition de la hyaluronidase par les molécules 1, 4, 5 et 6 sont équivalentes ou plus importantes que celle de la l'ascorbylpalmitate et la vitamine C. L'activité anti hyaluronidase des molécules décrites est donc supérieure à celle de l'acide ascorbique et du 6-ascorbyl palmitate.

La présente invention concerne donc des composés de formule générale I pour la prévention et/ou le traitement du vieillissement cutané ainsi que pour améliorer et /ou restaurer l'hydratation cutanée.

4.6.1 Protocole de la stimulation de la prolifération des cellules de la papille dermique in vitro

Des cellules humaines de la papille dermique (Promocell) sont cultivées et maintenues en culture à des passages précoces en milieu DMEM supplémenté avec 10% SVF. Elles sont ensuite ensemencées dans une plaque de 96-puits en milieu DMEM supplémenté de 10% SVF pendant 12 h. Le milieu de culture est alors remplacé par du milieu DMEM sans sérum puis par du DMEM supplémenté avec 1%SVF et les molécules 1, 2, 3 et 4 aux différentes concentrations testées. A l'issue d'une incubation de 60 h, la prolifération des cellules est évaluée par l'incorporation de BrdU.

4.6.2 Résultats du test de la stimulation de la prolifération des cellules de la papille dermique in vitro

Les diagrammes de la figure 1 annexée montrent l'augmentation de l'incorporation du BrdU ainsi que la stimulation de la prolifération cellulaire des cellules en présence des différentes molécules (en % du contrôle).

La molécule 1 stimule la prolifération des cellules de la papille dermique selon un effet dose avec la meilleure activité à la concentration de 12.5µM (169% prolifération). De même la molécule 4 induit une prolifération avec une stimulation de 113.9% à la concentration de 6.25µM. Les molécules 2 et 3 induisent également une prolifération des cellules mais de façon moins importante que les molécules 1 et 4.

La présente invention concerne également l'utilisation des composés de formule générale (I) pour stimuler la repousse des cheveux et/ou des poils.

4.7.1 Protocole de la stimulation de la croissance du follicule pileux humain

Des biopsies de la région occipitale d'un scalp humain sont obtenues à partir de déchet opératoire. Les follicules pileux sont isolés par micro-dissection sous loupe binoculaire. Ils sont individuellement mis en culture selon la technique de Philpott (Philpott MP et al, J Cell Sci. 97 :463-471, 1990). Les follicules pileux en phase anagène sont incubés dans des boîtes de culture de 24 puits en présence de milieu de William's supplémenté avec 10 µg/mL Insuline, 10 µg/mL Transferrine, 10 ng/mL Hydrocortisone, 1 mM Glutamax I, 100U/mL Pénicilline, 100 µg/mL Streptomycine, 250 ng/mL Amphotéricine B et en présence des molécules 3 ou 4 pendant 11 jours. Le milieu d'incubation est renouvelé régulièrement. L'allongement (en µm) de chaque follicule pileux est mesuré à 4 et 8 jours de culture à partir de capture d'images des follicules pileux.

4.7.2 Résultats du test de la stimulation de la croissance du follicule pileux humain

Les diagrammes de la figure 2 annexée montrent l'effet des molécules 3 et 4 sur l'allongement des follicules pileux après 4 jours et 8 jours d'incubation.

Le tableau suivant (Tableau 5) rapporte les pourcentages de croissance obtenus dans chaque condition de traitement et les pourcentages de stimulation de la croissance du cheveu obtenus avec les molécules 3 et 4 par rapport au contrôle.

Tableau 5 : Pourcentages de croissance et Pourcentages de stimulation de la croissance du cheveu

| Jours d'incubation | J4 | J8 | J4 | J8 |
|---|---|---|---|---|
| | % croissance | % croissance | % activité | % activité |
| Non traité | 44 | 77 | 0 | 0 |
| Molécule 3 | 52 | 85 | 25 | 18 |
| Molécule 4 | 51 | 84 | 19 | 12 |

Les molécules 3 et 4, à la concentration de 15μM, stimulent la croissance des cheveux de manière significative avec 25% et 19% de stimulation de la croissance respectivement, après 4 jours de culture.

La présente invention concerne également des composés de formule générale (I) pour stimuler la repousse des cheveux et/ou des poils.

4.8.1 Protocole de l'évaluation de la dégénérescence du bulbe pileux

La dégénérescence du follicule, en fin de cinétique après 11 jours de culture, est évaluée par observation des atteintes morphologiques du bulbe pileux (arrondissement, déformation). Le pourcentage d'apoptose est calculé par comptage du nombre de follicules pileux apoptotiques par rapport au nombre total de follicules.

4.8.2 Résultats de l'évaluation de la dégénérescence du bulbe pileux

Le tableau suivant (Tableau 6) montre l'effet de la molécule 4 sur la survie des follicules pileux après 11 jours d'incubation.

Tableau 6 : Survie des follicules pileux après 11 jours d'incubation

| Nombre de bulbes apoptotiques à J11 (Nombre de follicules totaux) | | | | |
|---|---|---|---|---|
| | | | Nbre | % d'apoptose |
| Contrôle | | | 9 (12) | 75 |
| Molécule 4 | | 15 μM | 6 (12) | 50 |

La molécule 4 a permis de limiter nettement la dégénérescence des cheveux (50% de cheveux apoptotiques versus 75% pour le contrôle). Ainsi, la molécule 4 aurait un effet anti-apoptose permettant de maintenir le cheveu en survie.

La présente invention concerne également des composés de formule générale (I) pour stimuler la repousse des cheveux et/ou des poils.

## Revendications

1. Composé de formule générale (I) suivante :

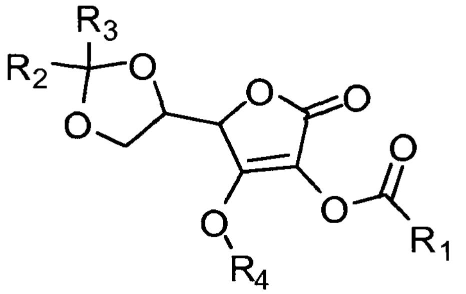

(I)

dans laquelle :

- $R_1$ représente une chaîne hydrocarbonée d'un acide gras insaturé de $C_{12}$ à $C_{24}$ comprenant au moins une insaturation, avantageusement 1 à 6, et de préférence 1 à 4 ; et
- $R_2$ et $R_3$ représentent indépendamment ou simultanément : un Hydrogène ou un $C_1$-$C_3$ alkyle ou un Phényle ; et
- $R_4$ : un atome d'hydrogène ou $COR_1$', avec $R_1$' représente une chaîne ,hydrocarbonée d'un acide gras insaturé de $C_{12}$ à $C_{24}$ comprenant au moins une insaturation, avantageusement 1 à 6, et de préférence 1 à 4.

**2.** Composé de formule générale (I) selon la revendication 1 **caractérisé en ce que** $R_2$ et $R_3$ représentent un $C_1$-$C_3$ alkyle.

**3.** Composé de formule générale (I) selon l'une des revendications 1 à 2 **caractérisé en ce que** $R_2$ et $R_3$ représentent un Méthyle.

**4.** Composé de formule générale (I) selon l'une des revendications 1 à 3 **caractérisé en ce que** :

$R_1$: représente la chaîne hydrocarbonée d'un acide gras insaturé de $C_{14}$ à $C_{18}$ comprenant de 1 à 3 insaturation (s) .

**5.** Composé de formule générale (I) selon l'une des revendications 1 à 4 **caractérisé en ce que** :

$R_1$' : représente la chaîne hydrocarbonée d'un acide gras insaturé de $C_{14}$ à $C_{18}$ comprenant de 1 à 3 insaturation (s) .

**6.** Composé de formule générale (I) selon l'une des revendications 1 à 5 **caractérisé en ce que** l'acide gras insaturé est choisi parmi le groupe constitué par : l'acide lauroléïque ($C_{12:1}$), l'acide myristoléïque ($C_{14:1}$), l'acide palmitoléïque ($C_{16:1}$), l'acide oléïque ($C_{18:1}$), l'acide ricinoléïque ($C_{18:1}$), l'acide gadoléïque ($C_{20:1}$), l'acide érucique ($C_{22:1}$), l'acide $\alpha$-linolénique ($C_{18:3}$), l'acide stéaridonique ($C_{18:4}$), l'acide eicosatriènoïque ($C_{20:3}$), l'acide eicosatetraènoïque ($C_{20:4}$), l'acide eicosapentaènoïque ($C_{20:5}$), l'acide docosapentaènoïque ($C_{22:5}$), l'acide docosahexaènoïque ($C_{22:6}$), l'acide tétracosapentaènoïque ($C_{24:5}$), l'acide tétracosahexaènoïque ($C_{24:6}$), l'acide linoléique ($C_{18:2}$), l'acide gamma-linolénique ($C_{18:3}$), l'acide eicosadiènoïque ($C_{20:2}$), l'acide dihomo-gamma-linolénique ($C_{20:3}$), l'acide arachidonique ($C_{20:4}$), l'acide docosatétraènoïque ($C_{22:2}$), l'acide docosapentaènoïque ($C_{22:5}$). l'acide adrénique ($C_{22:4}$) et l'acide calendique ($C_{18:3}$).

**7.** Composé de formule générale (I) selon l'une des revendications 1 à 6 **caractérisé en ce que** l'acide gras insaturé est choisi parmi le groupe constitué par : l'acide oléïque ($C_{18:1}$), l'acide linoléique ($C_{18:2}$), l'acide $\alpha$-linolénique ($C_{18:3}$) et l'acide $\gamma$-linolénique ($C_{18:3}$).

**8.** Composé de formule générale (I) selon l'une des revendications 1 à 7 **caractérisé en ce que** $R_4$ représente $COR_1$'.

**9.** Composé de formule générale (I) selon la revendication 8 **caractérisé en ce que** $R_1$ et $R_1$' peuvent être identiques ou différents.

**10.** Composé de formule générale (I) selon l'une des revendications 1 à 7 **caractérisé en ce que** $R_4$ représente un atome d'hydrogène.

**11.** Composé de formule générale (I) selon l'une des revendications 1 à 10 **caractérisé en ce qu'**il est choisi parmi:

• dioctadéca-9,12-diènoate de (9Z,9'Z,12Z,12'Z)-2-(2,2-diméthyl-1,3-dioxolan-4-yl)-5-oxo-2,5-dihydrofuran-3,4-diyle
• dioctadéca-9,12,15-triènoate de (9Z,9'Z,12Z,12'Z,15Z,15'Z)-2-(2,2-diméthyl-1,3-dioxolan-4-yl)-5-oxo-2,5-dihydrofuran-3,4-diyle
• dioctadéca-6,9,12-triènoate de (6Z,6'Z,9Z,9'Z,12Z,12'Z)-2-(2,2-diméthyl-1,3-dioxolan-4-yl)-5-oxo-2,5-dihydrofuran-3,4-diyle
• dioléate de (Z)-2-(2,2-diméthyl-1,3-dioxolan-4-yl)-5-oxo-2,5-dihydrofuran-3,4-diyle
• (9Z,12Z)-5-(2,2-dimethyl-l,3-dioxolan-4-yl)-4-hydroxy-2-oxo-2,5-dihydrofuran-3-yl octadeca-9,12-dienoate
• (9Z,12Z,15Z)-5-(2,2-dimethyl-1,3-dioxolan-4-yl)-4-hydroxy-2-oxo-2,5-dihydrofuran-3-yl octadeca-9,12,15-trienoate
• (6Z,9Z,12Z)-5-(2,2-dimethyl-1,3-dioxolan-4-yl)-4-hydroxy-2-oxo-2,5-dihydrofuran-3-yl octadeca-6,9,12-trienoate
• 5-(2,2-dimethyl-1,3-dioxolan-4-yl)-4-hydroxy-2-oxo-2,5-dihydrofuran-3-yl oleate.

**12.** Composé selon l'une des revendications 1 à 11 pour son utilisation en tant que médicament ou en tant que principe actif cosmétique.

**13.** Composé selon l'une des revendications 1 à 11 pour son utilisation en tant que principe actif dépigmentant, principe actif anti-âge, principe actif hydratant, principe actif anti-inflammatoire, principe actif stimulant la repousse du cheveu et/ou du poil, ou principe actif anti-oxydant.

**14.** Composition pour administration topique, **caractérisée en ce qu'**elle comprend au moins un composé de formule générale (I) tel que défini selon l'une des revendications 1 à 11.

**15.** Composition pour administration topique selon la revendication 14, **caractérisée en ce qu'**elle comprend au moins 0,01% à 10% en poids d'un composé de formule générale (I) et de préférence de 0,1% à 5% en poids d'un composé de formule générale (I).

**16.** Composition pour administration topique selon l'une des revendications 14 ou 15, **caractérisée en ce qu'**elle est destinée à la dépigmentation de la peau et/ou des cheveux et/ou des poils, au traitement et/ou à la prévention du vieillissement de la peau, à l'hydratation de l'épiderme, à la stimulation de la repousse du cheveu et/ou du poil, ou au traitement de l'inflammation de la peau.

**17.** Procédé de synthèse des composés de formule générale (I) selon l'une quelconque des revendications 1 à 11 par couplage d'un acide gras insaturé dont la fonction carboxylique se trouve sous forme activée, et d'un dérivé d'acide ascorbique de formule (II) suivante :

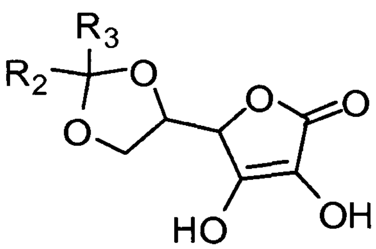

(II)

**18.** Procédé selon la revendication 17, **caractérisé en ce que** la réaction de couplage est réalisée à partir d'un acide gras insaturé dont la fonction acide carboxylique est activée sous forme d'un chlorure d'acide, en présence d'une base et éventuellement d'un auxiliaire de couplage.

## Patentansprüche

**1.** Verbindung der folgenden allgemeinen Formel (I):

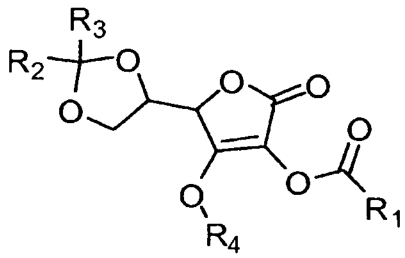

(I)

wobei:

- $R_1$ eine Kohlenwasserstoffkette einer ungesättigten Fettsäure mit $C_{12}$ bis $C_{24}$ repräsentiert, die mindestens eine Mehrfachbindung aufweist, günstigerweise 1 bis 6, und bevorzugt 1 bis 4; und
- $R_2$ und $R_3$ voneinander unabhängig oder gleichzeitig repräsentieren: ein Wasserstoff oder ein $C_1$-$C_3$-Alkyl oder ein Phenyl; und
- $R_4$: ein Wasserstoffatom oder $COR_1$', wobei $R_1$' eine Kohlenwasserstoffkette einer ungesättigten Fettsäure mit $C_{12}$ bis $C_{24}$ repräsentiert, die mindestens eine Mehrfachbindung aufweist, günstigerweise 1 bis 6, und bevorzugt 1 bis 4.

**2.** Verbindung der allgemeinen Formel (I) nach Anspruch 1, **dadurch gekennzeichnet, dass** $R_2$ und $R_3$ ein $C_1$-$C_3$-Alkyl repräsentieren.

**3.** Verbindung der allgemeinen Formel (I) nach einem der Ansprüche 1 bis 2, **dadurch gekennzeichnet, dass** $R_2$ und $R_3$ ein Methyl repräsentieren.

**4.** Verbindung der allgemeinen Formel (I) nach einem der Ansprüche 1 bis 3, **dadurch gekennzeichnet, dass**:

$R_1$: repräsentiert die Kohlenwasserstoffkette einer ungesättigten Fettsäure mit $C_{14}$ bis $C_{18}$, welche 1 bis 3 Mehrfachbindungen aufweist.

**5.** Verbindung der allgemeinen Formel (I) nach einem der Ansprüche 1 bis 4, **dadurch gekennzeichnet, dass**:

$R_1$': repräsentiert die Kohlenwasserstoffkette einer ungesättigten Fettsäure mit $C_{14}$ bis $C_{18}$, welche 1 bis 3 Mehrfachbindungen aufweist.

**6.** Verbindung der allgemeinen Formel (I) nach einem der Ansprüche 1 bis 5, **dadurch gekennzeichnet, dass** die ungesättigte Fettsäure ausgewählt ist aus der Gruppe bestehend aus: Lauroleinsäure ($C_{12:1}$), Myristoleinsäure ($C_{14:1}$), Palmitoleinsäure ($C_{16:1}$), Ölsäure ($C_{18:1}$), Rizinolsäure ($C_{18:1}$), Gadoleinsäure ($C_{20:1}$), Erucasäure ($C_{22:1}$), $\alpha$-Linolensäure ($C_{18:3}$), Stearidonsäure ($C_{18:4}$), Eicosatriensäure ($C_{20:3}$), Eicosatetraensäure ($C_{20:4}$), Eicosapentaensäure ($C_{20:5}$), Docosapentaensäure ($C_{22:5}$), Docosahexaensäure ($C_{22:6}$), Tetracosapentaensäure ($C_{24:5}$), Tetracosahexaensäure ($C_{24:6}$), Linolsäure ($C_{18:2}$), Gamma-Linolensäure ($C_{18:3}$), Eicosadiensäure ($C_{20:2}$), Dihomo-Gamma-Linolensäure ($C_{20:3}$), Arachidonsäure ($C_{20:4}$), Docosatetraensäure z22:2), Docosapentaensäure ($C_{22:5}$), Adrensäure ($C_{22:4}$) und Calendulasäure ($C_{18:3}$).

**7.** Verbindung der allgemeinen Formel (I) nach einem der Ansprüche 1 bis 6, **dadurch gekennzeichnet, dass** die ungesättigte Fettsäure ausgewählt ist aus der Gruppe bestehend aus: Ölsäure ($C_{18:1}$), Linolsäure ($C_{18:2}$), $\alpha$-Linolensäure ($C_{18:3}$) und $\gamma$-Linolensäure ($C_{18:3}$).

**8.** Verbindung der allgemeinen Formel (I) nach einem der Ansprüche 1 bis 7, **dadurch gekennzeichnet, dass** $R_4$ COR,' repräsentiert.

**9.** Verbindung der allgemeinen Formel (I) nach Anspruch 8, **dadurch gekennzeichnet, dass** $R_1$ und $R_1$' identisch oder verschieden sein können.

**10.** Verbindung der allgemeinen Formel (I) nach einem der Ansprüche 1 bis 7, **dadurch gekennzeichnet, dass** $R_4$ ein Wasserstoffatom repräsentiert.

**11.** Verbindung der allgemeinen Formel (I) nach einem der Ansprüche 1 bis 10, **dadurch gekennzeichnet, dass** sie ausgewählt ist aus:

- (9Z, 9'Z, 12Z, 12'Z)-2-(2,2-Dimethyl-1,3-dioxolan-4-yl)-5-oxo-2,5-dihydrofuran-3,4-diyl-Dioctadeca-9,12-dienoat
- (9Z, 9'Z, 12Z, 12'Z, 15Z, 15'Z)-2-(2,2-Dimethyl-1,3-dioxolan-4-yl)-5-oxo-2,5-dihydrofuran-3,4-diyl-Dioctadeca-9,12,15-trienoat
- (6Z, 6'Z, 9Z, 9'Z, 12Z, 12'Z)-2-(2,2-Dimethyl-1,3-dioxolan-4-yl)-5-oxo-2,5-dihydrofuran-3,4-diyl-Dioctadeca-6,9,12-trienoat
- (Z)-2-(2,2-Dimethyl-1,3-dioxolan-4-yl)-5-oxo-2,5-dihydrofuran-3,4-diyl-Dioleat
- (9Z, 12Z)-5-(2,2-Dimethyl-1,3-dioxolan-4-yl)-4-hydroxy-2-oxo-2,5-dihydrofuran-3-yl-Octadeca-9,12-dienoat
- (9Z, 12Z, 15Z)-5-(2,2-Dimethyl-1,3-dioxolan-4-yl)-4-hydroxy-2-oxo-2,5-dihydrofuran-3-yl-Octadeca-9,12,15-trienoat
- (6Z, 9Z, 12Z)-5-(2,2-Dimethyl-1,3-dioxolan-4-yl-4-hydroxy-2-oxo-2,5-dihydrofuran-3-yl-Octadeca-6,9,12-trienoat
- 5-(2,2-Dimethyl-1,3-dioxolan-4-yl)-4-hydroxy-2-oxo-2,5-dihydrofuran-3-yl-Oleat.

**12.** Verbindung nach einem der Ansprüche 1 bis 11 für die Verwendung als Medikament oder als kosmetischer Wirkstoff.

**13.** Verbindung nach einem der Ansprüche 1 bis 11 für die Verwendung als depigmentierender Wirkstoff, Anti-Aging-Wirkstoff, hydratisierender Wirkstoff, entzündungshemmender Wirkstoff, Wirkstoff zum Stimulieren des Wachstums des Kopfhaars und/oder der Körperbehaarung, oder antioxidativer Wirkstoff.

**14.** Zusammensetzung zur topischen Verabreichung, **dadurch gekennzeichnet, dass** die mindestens eine Verbindung der allgemeinen Formel (I) nach einem der Ansprüche 1 bis 11 umfasst.

**15.** Zusammensetzung zur topischen Verabreichung nach Anspruch 14,
**dadurch gekennzeichnet, dass** sie mindestens 0,01 bis 10 Gew.% einer Verbindung der allgemeinen Formel (I) und bevorzugt 0,1 bis 5 Gew.% einer Verbindung der allgemeinen Formel (I) umfasst.

**16.** Zusammensetzung zur topischen Verabreichung nach Anspruch 14 oder 15, **dadurch gekennzeichnet, dass** die zum Depigmentieren der Haut und/oder des Kopfhaars und/oder der Körperbehaarung, zur Behandlung und/oder Vorbeugung der Alterung der Haut, zur Hydratisierung der Epidermis, zur Stimulation des Wachstums des Kopfhaars und/oder der Körperbehaarung oder zur Behandlung von Entzündungen der Haut bestimmt ist.

**17.** Verfahren zur Synthese der Verbindungen der allgemeinen Formel (I) nach einem der Ansprüche 1 bis 11 durch Kopplung einer ungesättigten Fettsäure, deren Carboxylfunktion sich in aktiviertem Zustand befindet, und eines Ascorbinsäurederivats der folgenden Formel (II):

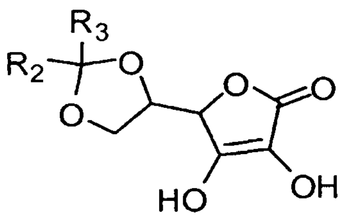

(II)

**18.** Verfahren nach Anspruch 17, **dadurch gekennzeichnet, dass** die Kopplungsreaktion ausgehend von einer ungesättigten Fettsäure, deren Carboxylsäurefunktion in Form eines Carbonsäurechlorids aktiviert ist, in Gegenwart einer Base und gegebenenfalls eines Kopplungshilfsmittels durchgeführt wird.

## Claims

**1.** A compound of general formula (I):

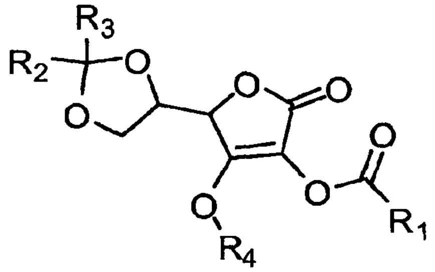

(I)

wherein:

- $R_1$ is a hydrocarbon chain of a $C_{12}$ to $C_{24}$ unsaturated fatty acid comprising at least one unsaturation, advantageously 1 to 6, and preferably 1 to 4; and
- $R_2$ and $R_3$ independently or simultaneously represent: a hydrogen or $C_1$-$C_3$ alkyl or a Phenyl; and
- $R_4$: a hydrogen atom or $COR_1'$, where $R_1'$ is a hydrocarbon chain of a $C_{12}$ to $C_{24}$ unsaturated fatty acid comprising at least one unsaturation, advantageously 1 to 6, and preferably 1 to 4.

**2.** The compound of general formula (I) according to claim 1, **characterized in that** $R_2$ and $R_3$ represent a $C_1$-$C_3$ alkyl.

**3.** The compound of general formula (I) according to one of claims 1 to 2, **characterized in that** $R_2$ and $R_3$ represent a Methyl.

**4.** The compound of general formula (I) according to one of claims 1 to 3 **characterized in that**:

$R_1$: represents the hydrocarbon chain of a $C_{14}$ to $C_{18}$ unsaturated fatty acid comprising 1 to 3 unsaturations.

**5.** The compound of general formula (I) according to one of claims 1 to 4 **characterized in that**:

$R_1'$ : represents the hydrocarbon chain of a $C_{14}$ to $C_{18}$ unsaturated fatty acid comprising 1 to 3 unsaturations.

**6.** The compound of general formula (I) according to one of claims 1 to 5 **characterized in that** the unsaturated fatty acid is chosen from the group composed of: lauroleic acid ($C_{12:1}$), myristoleic acid ($C_{14:1}$), palmitoleic acid ($C_{16:1}$), oleic acid ($C_{18:1}$), ricinoleic acid ($C_{18:1}$) ; gadoleic acid ($C_{20:1}$), erucic acid ($C_{22:1}$), $\alpha$-linolenic acid ($C_{18:3}$) ; stearidonic acid ($C_{18:4}$), eicosatrienoic acid ($C_{20:3}$), eicosatetraenoic acid ($C_{20:4}$), eicosapentaenoic acid ($C_{20:5}$), docosapentaenoic acid ($C_{22:5}$), docosahexaenoic acid ($C_{22:6}$), tetracosapentaenoic acid ($C_{24:5}$), tetracosahexaenoic acid ($C_{24:6}$), linoleic acid ($C_{18:2}$), gamma-linolenic acid ($C_{18:3}$), eicosadienoic acid ($C_{20:2}$) ; dihomo-gamma-linolenic acid ($C_{20:3}$), arachidonic acid ($C_{20:4}$), docosatetraenoic acid ($C_{22:2}$), docosapentaenoic acid ($C_{22:5}$), adrenic acid ($C_{22:4}$) and calendic acid ($C_{18:3}$).

**7.** The compound of general formula (I) according to one of claims 1 to 6 **characterized in that** the unsaturated fatty acid is chosen from the group composed of: oleic acid ($C_{18:1}$), linoleic acid ($C_{18:2}$), $\alpha$-linolenic acid ($C_{18:3}$) and $\gamma$-linolenic acid ($C_{18:3}$).

**8.** The compound of general formula (I) according to one of claims 1 to 7 **characterized in that** $R_4$ represents $COR_1'$.

**9.** The compound of general formula (I) according to claim 8, **characterized in that** $R_1$ and $R_1'$ may be the same or different.

**10.** The compound of general formula (I) according to one of claims 1 to 7 **characterized in that** $R_4$ is a hydrogen atom.

**11.** The compound of general formula (I) according to one of claims 1 to 10 **characterized in that** it is chosen from among:

- dioctadeca-9,12-dienoate of (9Z,9'Z,12Z,12'Z)-2-(2,2-dimethyl-1,3-dioxolan-4-yl)-5-oxo-2,5-dihydrofuran-3,4-diyl
- dioctadeca-9,12,15-trienoate of (9Z,9'Z,12Z,12'Z,15Z,15'Z)-2-(2,2-dimethyl-1,3-dioxolan-4-yl)-5-oxo-2,5-dihydrofuran-3,4-diyl

• dioctadeca-6,9,12-trienoate of (6Z,6'Z,9Z,9'Z,12Z,12'Z)-2-(2,2-dimethyl-1,3-dioxolan-4-yl)-5-oxo-2,5-dihydrofuran-3,4-diyl
• dioleate of (Z)-2-(2,2-dimethyl-l,3-dioxolan-4-yl)-5-oxo-2,5-dihydrofuran-3,4-diyl
• (9Z,12Z)-5-(2,2-dimethyl-l,3-dioxolan-4-yl)-4-hydroxy-2-oxo-2,5-dihydrofuran-3-yl octadeca-9,12-dienoate
• (9Z,12Z,15Z)-5-(2,2-dimethyl-1,3-dioxolan-4-yl)-4-hydroxy-2-oxo-2,5-dihydrofuran-3-yl octadeca-9,12,15-trienoate
• (6Z,9Z,12Z)-5-(2,2-dimethyl-1,3-dioxolan-4-yl)-4-hydroxy-2-oxo-2,5-dihydrofuran-3-yl octadeca-6,9,12-trienoate
• 5-(2,2-dimethyl-1,3-dioxolan-4-yl)-4-hydroxy-2-oxo-2,5-dihydrofuran-3-yl oleate.

**12.** The compound of one of claims 1 to 11 for use thereof as medication or as cosmetic active ingredient.

**13.** The compound according to one of claims 1 to 11 for use thereof as depigmenting active ingredient, anti-age active ingredient, hydrating active ingredient, anti-inflammatory active ingredient, active ingredient for stimulating re-growth of head and/or body hair, or anti-oxidant active ingredient.

**14.** A composition for topical administration, **characterized in that** it comprises at least one compound of general formula (I) such as defined in one of claims 1 to 11.

**15.** The composition for topical administration according to claim 14, **characterized in that** it comprises at least 0.01% to 10% by weight of a compound of general formula (I) and preferably from 0.1% to 5% by weight of a compound of general formula (I).

**16.** The composition for topical administration according to one of claims 14 or 15, **characterized in that** it is intended for depigmenting the skin and/or head hair and/or body hair, for the treatment and/or prevention of skin ageing, for hydrating the epidermis, for stimulating re-growth of head and/or body hair, or for the treatment of skin inflammation.

**17.** A method for synthesizing compounds of general formula (I) according to any of claims 1 to 11 by coupling an unsaturated fatty acid whose carboxylic function is in activated form, with a derivative of ascorbic acid of following formula (II):

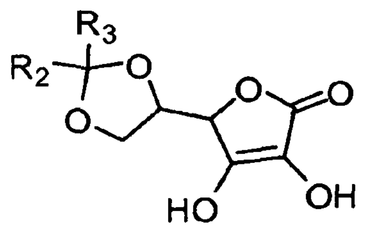

(II)

**18.** The method according to claim 17, **characterized in that** the coupling reaction is performed starting from an unsaturated fatty acid whose carboxylic acid function is activated in the form of an acid chloride, in the presence of a base and optionally a coupling auxiliary.

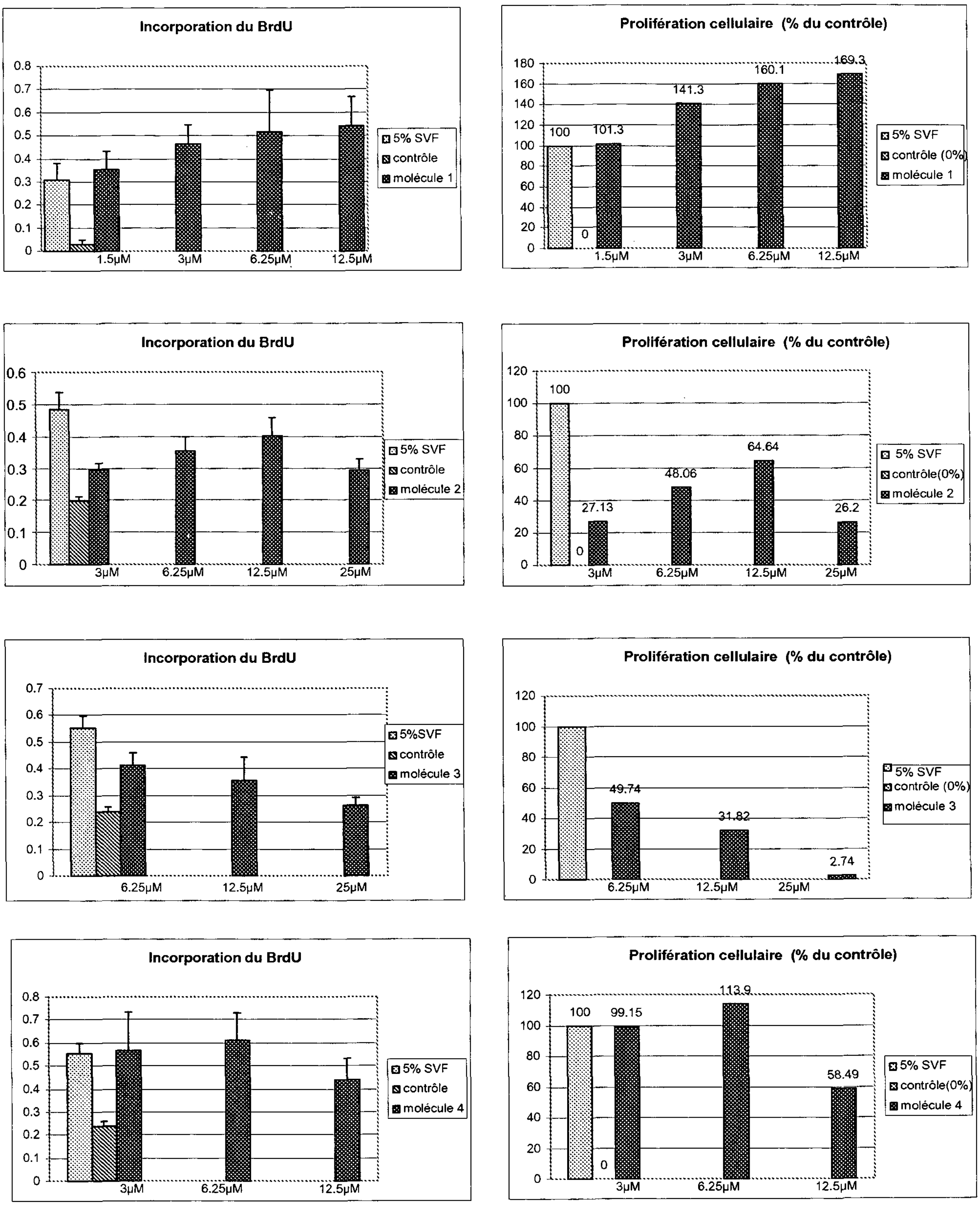
Incorporation du BrdU
0.8
0.7
0.6
0.5
0.4
0.3
0.2
0.1
0
1.5µM
3µM
6.25µM
12.5µM
5% SVF
contrôle
molécule 1
Prolifération cellulaire (% du contrôle)
180
160
140
120
100
80
60
40
20
0
100
101.3
141.3
160.1
169.3
1.5µM
3µM
6.25µM
12.5µM
5% SVF
contrôle (0%)
molécule 1
Incorporation du BrdU
0.6
0.5
0.4
0.3
0.2
0.1
0
3µM
6.25µM
12.5µM
25µM
5% SVF
contrôle
molécule 2
Prolifération cellulaire (% du contrôle)
120
100
80
60
40
20
0
100
27.13
48.06
64.64
26.2
3µM
6.25µM
12.5µM
25µM
5% SVF
contrôle(0%)
molécule 2
Incorporation du BrdU
0.7
0.6
0.5
0.4
0.3
0.2
0.1
0
6.25µM
12.5µM
25µM
5%SVF
contrôle
molécule 3
Prolifération cellulaire (% du contrôle)
120
100
80
60
40
20
0
49.74
31.82
2.74
6.25µM
12.5µM
25µM
5% SVF
contrôle (0%)
molécule 3
Incorporation du BrdU
0.8
0.7
0.6
0.5
0.4
0.3
0.2
0.1
0
3µM
6.25µM
12.5µM
5% SVF
contrôle
molécule 4
Prolifération cellulaire (% du contrôle)
120
100
80
60
40
20
0
100
99.15
113.9
58.49
3µM
6.25µM
12.5µM
5% SVF
contrôle(0%)
molécule 4

**Figure 1**

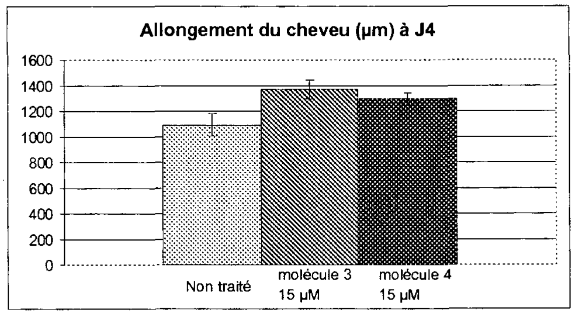
Allongement du cheveu (µm) à J4
1600
1400
1200
1000
800
600
400
200
0
Non traité
molécule 3 15 µM
molécule 4 15 µM

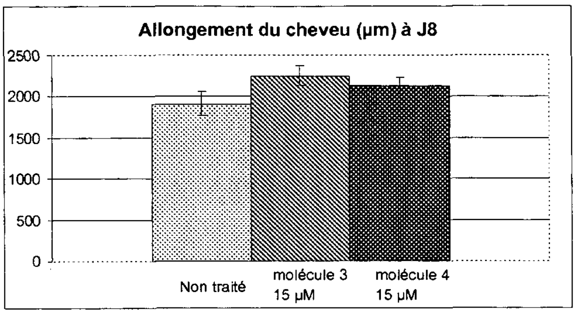
Allongement du cheveu (µm) à J8
2500
2000
1500
1000
500
0
Non traité
molécule 3 15 µM
molécule 4 15 µM

**Figure 2**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Littérature non-brevet citée dans la description


- **REN J ; CHUNG SH.** *J Agric Food Chem.,* 2007, vol. 55, 5073-80
- **CALDER PC.** *Lipids,* 2001, vol. 36, 1007-24
- **MONPOINT S ; GUILLOT B ; TRUCHETET F et al.** Acides gras essentiels en dermatologie. *Ann Dermatol Vénéréol,* 1992, vol. 119, 233-239
- **REDOULES, D. ; TARROUX, R. ; ASSALIT, M.F. ; PERIÉ, J.J.** Caracterization and assay of five enzymatic activities in the stratum corneum using tape-stripping. *Skin Pharmacol. Appl. Skin Physiol.,* 1999, vol. 12, 182-192
- **FISHER et al.** *N. Engl. J. Med.,* 1997, vol. 333, 1419
- **SHAPIRO SD.** *Current Opinion in Cell Biology,* 1998, vol. 10, 602
- **NAGASE ; WOESSNER.** *J. Biol. Chem.,* 1999, vol. 274, 21491
- **KÄHARI VM ; SAARIALHO-KERE U.** *Exp. Dermatol.,* 1997, vol. 6, 199
- **STERN R ; MAIBACH HI.** Hyaluronan in skin: aspects of aging and its pharmacologic modulation. *Clin Dermatol,* 2008, vol. 26, 106-22
- **FRANCESCHI et al.** *Mechanisms of ageing and development,* 2006, 92
- **MOCCHEGIANI et al.** *Biomed Central,* 2004, vol. 1, 5
- **LICASTRO et al.** *Neurobiol. Aging,* 2006
- **BISSETT DL.** *J Cosmet Dermatol.,* Décembre 2006, vol. 5 (4), 309-15
- **HAYFLICK.** *J Invest Dermatol.,* 1979, vol. 73, 8-14
- **VANDESOMPELE et al.** *Genome Biol. 3: RESEARCH00,* 2002, 34
- **VANDESOMPELE J et al.** *Genome Biol. 3: RESEARCH00,* 2002, 34
- **DELPECH B.** *Analytical Biochemistry,* 1985, vol. 149, 555-565
- **ROBERT STERN.** *Analytical Biochemistry,* 1997, vol. 251, 263-269
- **A. BOTZKI.** *JBC,* vol. 279 (44), 45990-450007
- **PHILPOTT MP et al.** *J Cell Sci,* 1990, vol. 97, 463-471